# FASCICULE DE BREVET EUROPEEN

(11) **EP 2 916 808 B1**
(45) Date de publication et mention de la délivrance du brevet: **26.06.2019**
(21) Numéro de dépôt: 13803123.2
(22) Date de dépôt: 08.11.2013
(51) Int. Cl.: A61K 8/365, A61Q 5/04, A61K 8/42, A61K 8/43, A61K 8/44

(54) **COMPOSITION COMPRENANT UN DERIVE DICARBONYLE ET PROCEDE DE LISSAGE DES CHEVEUX A PARTIR DE CETTE COMPOSITION**
ZUSAMMENSETZUNG MIT EINEM DICARBONYLDERIVAT UND HAARGLÄTTUNGSVERFAHREN UNTER VERWENDUNG DIESER ZUSAMMENSETZUNG
COMPOSITION COMPRISING A DICARBONYL DERIVATIVE AND METHOD FOR STRAIGHTENING THE HAIR USING THIS COMPOSITION

(30) Priorité: 09.11.2012 FR 1260681
(43) Date de publication de la demande: 16.09.2015
(73) Titulaire: L'Oréal, 75008 Paris (FR)
(72) Inventeur: DAUBRESSE, Nicolas, F-78170 La Celle Saint Cloud (FR)
(74) Mandataire: Leray, Noelle
(86) Numéro de dépôt international: PCT/FR2013/052691
(87) Numéro de publication internationale: WO 2014/072658

(56) Documents cités:
- EP-A1- 0 711 543
- WO-A1-2007/135299
- WO-A1-2012/105985
- WO-A2-2007/135297
- WO-A2-2011/104282
- WO-A2-2012/010351
- FR-A1- 2 070 232
- DATABASE GNPD [Online] MINTEL; février 2012 (2012-02), "Moroccan Relaxing Treatment with Argan Oil", XP002703715, Database accession no. 1692744

## Description

La présente invention concerne une composition cosmétique, notamment capillaire, à base d'un ou de plusieurs dérivés dicarbonylés particuliers ainsi qu'un procédé de lissage des fibres kératiniques, notamment des cheveux, à partir de cette composition.

Dans le domaine capillaire, les consommateurs souhaitent disposer de compositions permettant d'apporter un changement temporaire à leur chevelure, et ce en visant une bonne tenue de l'effet réalisé. En général, on désire que le changement persiste aux shampooings pendant au minimum 15 jours, voire plus selon la nature dudit changement.

Des traitements existent déjà pour modifier la couleur ou la forme des cheveux ainsi que, dans une certaine mesure, la texture des cheveux. L'un des traitements connus pour modifier la texture des cheveux consiste en l'association de chaleur et d'une composition comprenant du formol. Ce traitement est spécialement efficace pour conférer un meilleur aspect aux cheveux abîmés, et/ou pour traiter les cheveux longs et les cheveux bouclés.

On associe l'action du formol à sa capacité de réticuler les protéines par réaction sur ses sites nucléophiles. La chaleur utilisée peut être celle du fer (pince plate ou fer à friser), dont la température peut atteindre en général 200°C ou plus. Toutefois, on cherche de plus en plus à éviter l'emploi de telles substances, qui peuvent se révéler agressives pour le cheveu et les autres matières kératiniques.

Il a ainsi été proposé, par la demande WO2011/104282, un nouveau procédé pour lisser de manière semi-permanente les cheveux, consistant à appliquer une solution d'alphacétoacide sur le cheveu pendant 15 à 120 minutes, puis à sécher et enfin à lisser au fer, à une température d'environ 200°C, la chevelure. L'alphacétoacide employé est de préférence l'acide glyoxylique.

Le document WO2012/105985 décrit un procédé de lissage consistant à appliquer une composition alcaline comprenant une base, puis, après séchage des cheveux, à appliquer une composition acide de pH inférieur à 1,5 comprenant un composé qui peut être l'acide glyoxylique ou un amide glyoxylique, ces applications étant suivies d'un passage de fer.

Toutefois, on a constaté que l'utilisation de l'acide glyoxylique pouvait engendrer quelques limitations importantes; en particulier, à forte concentration il peut ne pas être bien toléré, en particulier quand le cuir chevelu est sensible et/ou irrité. Sa volatilité, amplifiée par l'utilisation de chaleur (fer), peut également poser problème. Par ailleurs, les formulations cosmétiques à pH acide peuvent altérer les cheveux et/ou en altérer la couleur.

Il est déjà connu d'utiliser des esters d'acide glyoxylique dans des compositions capillaires, en particulier dans des compositions de coloration des cheveux tel que décrit dans le document DE19859722 et dans des compositions réductrices tel que décrit dans le document DE19860239.

L'efficacité de ces composés n'est cependant pas encore suffisante.

Le but de l'invention est de développer une composition de lissage/défrisage stable dans le temps et qui permet de lisser/défriser et/ou de réduire le volume les cheveux de façon efficace et rémanente en limitant la dégradation des cheveux tout en conservant un confort au moment de l'application pour l'utilisateur de la composition mais aussi pour le coiffeur qui l'applique.

Ainsi, un objet de la présente invention est un procédé de lissage des fibres kératiniques tel que défini dans la revendication 1.

La composition cosmétique utilisée dans le procédé de l'invention comprend :
- un ou plusieurs composés dicarbonylés répondant à la formule (I) suivante et/ou leurs dérivés et/ou leurs hydrates et/ou leurs sels: 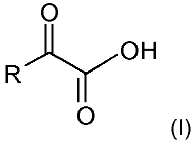 formule (I) dans laquelle
   R représente un atome ou groupe choisi parmi i) hydrogène, ii) carboxy -C(O)OH, iii) alkyle linéaire ou ramifié en C₁-C₆ éventuellement substitué de préférence par au moins un radical hydroxy -OH, carboxy -C(O)-OH ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyl et vii) un radical imidazolylméthyle et ses tautomères tel que 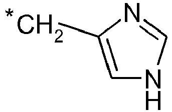 avec * représentant la partie reliée au reste de la molécule,
- un ou plusieurs dérivés aminogènes choisis parmi les composés de formule **(II)** suivante, leurs sels, leurs tautomères ou leurs hydrates : 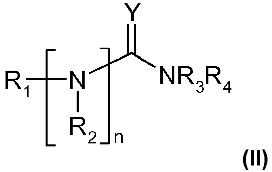 formule **(II)** dans laquelle :
- **Y** représente un atome ou groupe choisi parmi i) oxygène, ii) soufre, iii) N-R' ; de préférence Y représente un atome d'oxygène ou N-R', plus préférentiellement Y = O ;
- **R'** représentant un atome ou groupe choisi parmi i) hydrogène, ii) un radical alkyle ou alcényle en C₁-C₅, linéaire ou ramifié, cyclique ou acyclique, ce radical étant éventuellement substitué par un ou plusieurs radicaux choisis parmi les radicaux : hydroxyle, (di)(C₁-C₆)(alkyl)amino tel que diméthylamino, carboxyle, halogène, aryle en C₆-C₁₈, carboxamide et N-méthylcarboxamide ; de préférence R' représente un atome d'hydrogène ou un groupe (C₁-C₆)alkyle éventuellement substitué par un ou plusieurs radicaux (di)(C₁-C₆)(alkyl)amino et/ou carboxyle ;
- **n** = 0 ou 1, de préférence 1,
- **R₁**, **R₂**, **R₃** et **R₄**, identiques ou différents, représentent un i) atome d'hydrogène, une chaîne hydrocarbonée en C₁-C₃₀, linéaire ou ramifiée, saturée ou insaturée, éventuellement substituée, éventuellement interrompue et/ou éventuellement terminée à l'une ou deux de ses extrémités par un ou plusieurs groupes divalents ou leur combinaisons choisis parmi :
   ▪ -N(R)-, -N⁺(R)(R')-, -O-, -S-, -C(O)-, -S(O)-, -S(O)₂- avec R, R', identiques ou différents, sont choisis parmi un hydrogène, un radical alkyle en C₁-C₄, hydroxy(C₁-C₄)alkyle et (di)(C₁-C₄)(alkyl)aminoalkyle ; préférentiellement par un ou plusieurs groupes divalents ou leur combinaisons choisis parmi : -N(R)-, -O-, et - C(O)-,
   ▪ -N⁺(R)(R')-, -O-, -S-, -C(O)-, -S(O)-, -S(O)₂-
   ▪ un radical (hétéro)cycloalkyle éventuellement substitué, ou (hétéro)aryle éventuellement substitué ;
   ou alors
- **R₁** et **R₂** et/ou **R₃** et **R₄** forment ensemble avec l'atome d'azote qui les portent un groupe hétérocycloalkyle éventuellement substitué ou un groupe hétéroaryle éventuellement substitué ;
   ou alors
- **R₁** et **R₃** forment ensemble avec l'atome d'azote qui les portent un groupe hétérocycloalkyle éventuellement substitué.

Sauf mention contraire préalable :
Les radicaux « (hétéro)*aryle* », ou la partie « *(hétéro)aryle* » d'un radical lorsqu'ils sont éventuellement substitués, lesdits radicaux peuvent alors être substitués sur un atome de carbone, par un atome ou groupe choisi parmi : i) alkyle en C₁-C₁₆, de préférence en C₁-C₈, éventuellement substitué par un ou plusieurs radicaux choisis parmi les radicaux hydroxyle, alkoxy en C₁-C₂, (poly)-hydroxyalkoxy en C₂-C₄, acylamino, amino substitué par deux radicaux alkyle, identiques ou différents, en C₁-C₄, éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ; ii) halogène; iii) hydroxy ; iv) alkoxy en C₁-C₂ ; v) (poly)-hydroxyalkoxy en C₂-C₄ ; vi) amino ; vii) héterocycloalkyle à 5 ou 6 chaînons ; viii) hétéroaryle à 5 ou 6 chaînons éventuellement cationique, préférentiellement imidazolium, et éventuellement substitué par un radical (C₁-C₄) alkyle, préférentiellement méthyle ; ix) amino substitué par un ou deux radicaux alkyle, identiques ou différents, en C₁-C₆ éventuellement porteurs d'au moins un groupement hydroxy, amino éventuellement substitué par un ou deux radicaux alkyle en C₁-C₃ éventuellement substitués, x) acylamino (-N(R)-C(O)-R') dans lequel le radical R est un atome d'hydrogène, un radical alkyle en C₁-C₄ éventuellement porteur d'au moins un groupement hydroxy et le radical R' est un radical alkyle en C₁-C₂ ; xi) carbamoyle ((R)₂N-C(O)-) dans lequel les radicaux R, identiques ou non, représentent un atome d'hydrogène, un radical alkyle en C₁-C₄ éventuellement porteur d'au moins un groupement hydroxyle ; xii) alkylsulfonylamino (R'-S(O)₂-N(R)-) dans lequel le radical R représente un atome d'hydrogène, un radical alkyle en C₁-C₄ éventuellement porteur d'au moins un groupement hydroxy et le radical R' représente un radical alkyle en C₁C₄, un radical phényle ; xiii) aminosulfonyle ((R)₂N-S(O)₂-) dans lequel les radicaux R, identiques ou non, représentent un atome d'hydrogène, un radical alkyle en C₁-C₄ éventuellement porteur d'au moins un groupement hydroxyle, xiv) carboxy sous forme acide ou salifiée (de préférence avec un métal alcalin ou un ammonium, substitué ou non) ; xv) cyano ; xvi) nitro ou nitroso ; xvii) polyhalogénoalkyle, préférentiellement le trifluorométhyle ;
Les radicaux « *(hétéro)cyclique* » ou « *(hétéro)cycloakyle* » lorsqu'ils sont éventuellement substitués peut être substitués par au moins un atome ou groupe choisi parmi : i) hydroxy ; ii) alkoxy en C₁-C₄, (poly)hydroxyalkoxy en C₂-C₄ ; iii) alkyle en C₁-C₄ ; iv) alkylcarbonyl-amino (R-C(O)-N(R')-) dans lequel le radical R' est un atome d'hydrogène, un radical alkyle en C₁-C₄ éventuellement porteur d'au moins un groupement hydroxyle et le radical R est un radical alkyle en C₁-C₂, amino éventuellement substitué par un ou deux groupements alkyle identiques ou différents en C₁-C₄ eux-mêmes éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ; v) alkylcarbonyloxy (R-C(O)-O-) dans lequel le radical R est un radical alkyle en C₁-C₄, groupement amino éventuellement substitué par un ou deux groupements alkyle identiques ou différents en C₁-C₄ eux-mêmes éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ; vi) carboxy sous forme salifié ou non, ou alkoxycarbonyle (R-G-C(O)-) dans lequel le radical R est un atome d'hydrogène ou un radical alkoxy en C₁-C₄, G est un atome d'oxygène, ou un groupement amino éventuellement substitué par un groupement alkyle en C₁-C₄ lui-même éventuellement porteurs d'au moins un groupement hydroxyle, ledit radical alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ; vii) halogène ;
Les radicaux « *(hétéro)cyclique* » ou « *(hétéro)cycloalkyle* », ou une partie non aromatique d'un radical (hétéro)aryle, lorsqu'ils sont éventuellement substitués peuvent être également substitué par un ou plusieurs groupements oxo ;
Une chaîne hydrocarbonée est « *insaturée* » lorsqu'elle comporte une ou plusieurs liaisons doubles et/ou une ou plusieurs liaisons triples, conjuguées ou non ;
Un radical « *aryle* » représente un groupement carboné mono ou polycyclique, condensé ou non, comprenant de 6 à 22 atomes de carbone, et dont au moins un cycle est aromatique ; préférentiellement le radical aryle est un phényle, biphényle, naphtyle, indényle, anthracényle, ou tétrahydronaphtyle ;
Un « *radical hétéroaryle* » représente un groupement mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tetrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
Un « *radical hétérocyclique* » ou « *hétérocycloalkyle* » est un radical pouvant contenir une ou plusieurs insaturations mais non aromatique, mono ou polycyclique, condensé ou non, contenant de 5 à 22 chaînons, comportant de 1 à 6 hétéroatomes choisis parmi l'atome d'azote, d'oxygène, de soufre et de sélénium tel que morpholino, pipéridino, pipérazino, ou tétrahydrofuryle, pyrrolidyle;
Un « *radical cycloalkyle* » est un radical hydrocarboné pouvant contenir une ou deux insaturations mais non aromatique, mono ou polycyclique, condensé ou non, contenant de 5 à 22 chaînons tel que cyclohéxyle, cyclopentyle ou cyclobutyle ;
Un « *radical alkyle* » est un radical hydrocarboné en C₁-C₂₀, linéaire ou ramifié, de préférence en C₁-C₈ tel que méthyle ou éthyle ;
L'expression « *éventuellement substitué* » attribué au radical « *alkyle* », « *alkylène* », « *alkénylène* », ou à « *une chaine hydrocarbonée* » sous entend que ledit radical peut être substitué par un ou plusieurs radicaux choisis parmi les atomes d'halogène, les radicaux, i) hydroxyle, ii) alkoxy en C₁-C₄, iii) acylamino, iv) amino éventuellement substitué par un ou deux radicaux alkyle, identiques ou différents, en C₁-C₄, lesdits radicaux alkyles pouvant former avec l'atome d'azote qui les portent un hétérocycle comprenant de 5 à 7 chaînons, comprenant éventuellement un autre hétéroatome différent ou non de l'azote ; v) ou un groupement ammonium quaternaire -N⁺R'R"R'", M⁻ pour lequel R', R", R'", identiques ou différents représentent un atome d'hydrogène, ou un groupement alkyle en C₁-C₄, ou alors -N⁺R'R"R'" forme un hétéroaryle tel que imidazolium éventuellement substitué par un groupement C₁-C₄ alkyle, et M⁻ représente le contre-ion de l'acide organique, minéral ou de l'halogénure correspondant, vi) oxo ;
Un « *radical alkoxy* » est un radical alkyle-oxy pour lequel le radical alkyle est un radical hydrocarboné, linéaire ou ramifié, en C₁-C₁₆ préférentiellement en C₁-C₈ ;
Lorsque le groupe alkoxy est éventuellement substitué, cela sous entend que le groupe alkyle est éventuellement substitué tel que défini supra.

L'invention a pour objet un procédé de lissage des fibres kératiniques, notamment des cheveux, qui comprend l'application sur les cheveux de la composition décrite précédemment suivi d'une étape de lissage au moyen d'un fer de lissage à une température d'au moins 150°C, de préférence variant de 150 à 250°C.

La composition utilisée dans l'invention est stable. La composition utilisée dans l'invention et le procédé de traitement des fibres kératiniques la mettant en oeuvre permettent un bon lissage des fibres kératiniques en limitant la dégradation de ces fibres kératiniques, même lorsque l'application de la composition est suivie par un traitement thermique, notamment au moyen de fer de lissage des cheveux et présentent une qualité d'usage appréciée, notamment sans vaporisation excessive de la composition au moment du lissage. La composition utilisée dans l'invention et le procédé de traitement des fibres kératiniques selon l'invention permettent aussi de limiter le changement de la couleur des fibres, ainsi que les problèmes de casse des fibres telles que les cheveux. La composition utilisée dans l'invention et le procédé de l'invention vont en outre améliorer les propriétés physiques des cheveux, en réduisant l'effet de frisottis de façon durable.

Dans ce qui suit l'expression « au moins un » est équivalente à l'expression « un ou plusieurs ».

De préférence, la composition selon l'invention ne comprend ni agent colorant ni agent réducteur.

Par « agents colorants », on entend selon la présente invention des agents de coloration des fibres kératiniques tels que les colorants directs, les pigments ou les précurseurs de colorant d'oxydation (bases et coupleurs). S'ils sont présents, leur teneur ne dépasse pas 0,001 % en poids par rapport au poids total de la composition. En effet, à une telle teneur, seule la composition serait teintée, c'est-à-dire qu'on n'observerait pas d'effet de coloration des fibres kératiniques.

On rappelle que les précurseurs de colorants d'oxydation, bases d'oxydation et coupleurs, sont des composés peu ou non colorés qui par une réaction de condensation en présence d'un agent oxydant, donnent une espèce colorée. Quant aux colorants directs, ces composés sont colorés et présentent une certaine affinité pour les fibres kératiniques. Par « agent réducteur », on entend selon la présente invention un agent capable de réduire les liaisons disulfures des cheveux, tel que les composés choisis parmi les thiols, les sulfites alcalins, les hydrures, les phosphines.

Dans la présente invention, les composés dicarbonylés de formule **(I)** ou leurs dérivés peuvent se présenter sous forme libre mais aussi sous leurs formes hydrates ou sous forme de leurs sels, de préférence sous forme libre ou d'hydrates. Par « *dérivés* » des composés dicarbonylés de formule **(l)**, on entend les esters du ou des groupes carboxy, les amides du ou des groupes carboxy, les (thio)acétals et hémi(thio)acétals de la ou des fonctions carbonyles des composés de formule **(I)** sous forme libre ou éventuellement sous forme de sels ou d'hydrates, de préférence sous forme libre ou d'hydrates.

Les esters et amides peuvent être synthétisés à partir des procédés d'estérification ou d'amidification classiques à partir des acides correspondants bien connus par l'homme du métier.

Les esters sont par exemple obtenus à partir des acides de formule (I) et d'un mono ou polyalcool.

Par « *mono ou poly alcool* », on entend un composé organique comprenant un groupe hydroxy (monoalcool) ou au moins deux groupes hydroxy (polyalcool ou polyol) ; ledit composé organique hydroxylé pouvant être aliphatique, acyclique, linéaire ou ramifié, ou (hétéro)cyclique, tels que les sucres (mono ou polysaccharides) ou les sucres alcools. Plus particulièrement, le polyalcool comprend de 2 à 100 groupes hydroxy; et préférentiellement de 2 à 20 groupes hydroxy ; encore plus préférentiellement de 2 à 10 groupes hydroxy, mieux 2 ou 3 groupes hydroxy.

De préférence le mono ou polyalcool est choisi parmi le méthanol, l'éthanol, le propanol, l'isopropanol, le butanol, l'hexanol, l'éthylène glycol, le glycérol, la dihydroxyacétone, le glucose, le sorbitol, le menthol.

Les amides sont par exemple obtenus à partir des acides de formule (I) et d'une mono ou polyamine organique.

Par « mono ou polyamine », on entend un composé organique comprenant un groupe amino(monoamine) ou au moins deux (et de préférence de 2 à 100, mieux de 2 à 20) groupes amino; le dit composé organique pouvant être aliphatique, acyclique, linéaire ou ramifié ou (hétéro)cyclique.

Par groupe « amino » on entend un groupe amine primaire -NH₂, ou secondaire >NH.

De préférence la mono ou polyamine est aliphatique.

Cette amine est de préférence choisie parmi la méthylamine, l'éthylamine, la propylamine, l'isopropylamine, la butylamine, l'hexylamine, la monoéthanolamine, la monopropanolamine, la propane-1,2,3-triamine et la diaminoacétone.

Les (thio)acétals et hém(thio)iacétals des acides de formule (I) peuvent par exemple être obtenus à partir de la réaction d'alcools pour les acétals ou hémiacétals ou de thiols pour les thioacétals ou hémithioacétals sur des formes bloquées des acides puis hydrolyse. Les alcools peuvent être les mêmes que ceux cités pour les esters. Les thiols peuvent être des équivalents (appelés mono ou polythiols) aux mono ou polyalcools cités supra à ceci près que la ou les fonctions hydroxy desdits mono ou polyalcools sont remplacées par une ou des fonctions thiols SH des mono ou polythiols. Les acétals ou thioacétals peuvent également être des (thio)acétals cycliques.

On peut en particulier citer l'acide diméthoxy acétique, l'acide diéthoxy acétique, l'acide 1,3-dioxane 2 carboxylique, l'acide 1,3-dioxolane 2-carboxylique.

Les sels peuvent être des sels issus de l'interaction des composés de formule (I) avec des acides ou des bases, les acides ou bases pouvant être de nature organique ou minérale.

De préférence les sels sont des sels issus de l'interaction des composés de formule (I) avec des bases. On citera en particuliers les sels de métaux alcalins alcalins ou alcalino terreux et en particulier les sels de sodium.

De préférence, le ou les dérivés dicarbonylés répondant à la formule (I) et/ou leurs dérivés et/ou leurs hydrates et/ou leurs sels sont choisis parmi les dérivés dicarbonylés répondant à la formule (I) dans laquelle R représente i) un atome hydrogène ou ii) un groupe alkyle linéaire ou ramifié en C₁-C₆ éventuellement substitué par un groupe carboxy.

De préférence encore, ils sont choisis parmi l'acide glyoxylique, l'acide pyruvique, l'un de leurs dérivés et leurs hydrates ou leurs sels et encore préférentiellement parmi l'acide glyoxylique, ses dérivés et les formes hydrate de ces composés.

Préférentiellement le ou les composés dicarbonylés de formule (I) de l'invention sont choisis parmi l'acide glyoxylique et ses dérivés et les formes hydrate de ces composés.

On peut citer l'acide glyoxylique ainsi que sa forme hydrate (HO)₂CH-C(O)-OH tel que par exemple l'acide glyoxylique en solution aqueuse à 50 % vendu par la société MERCK.

En tant que dérivés d'acide glyoxylique, on peut citer les esters d'acide glyoxylique, les amides d'acide glyoxylique, les (thio)acétals et hémi(thio)acétals d'acide glyoxylique, les (thio)acétals et hémi(thio)acétals d'ester d'acide glyoxylique.

Les esters d'acide glyoxylique sont par exemple obtenus à partir de l'acide glyoxylique et d'un mono ou polyalcool, notamment ceux cités précédemment.

De préférence le mono ou polyalcool est choisi parmi le méthanol, l'éthanol, le propanol, l'isopropanol, le butanol, l'hexanol, l'éthylène glycol, le glycérol, la dihydroxyacétone, le glucose, le sorbitol, le menthol.

On peut en particulier citer à titre d'esters le glyoxylate de méthyle, le glyoxylate d'éthyle, le glyoxylate de glycérol, le glyoxylate de dihydroxyacétone, le diglyoxylate ou triglyoxylate de glycérol, les mono, di ou tri glyoxylate de sorbitol, les mono, di ou tri glyoxylate de glucose, le glyoxylate de menthyle, leurs acétals, hémiacétals, hydrates. Les amides d'acide glyoxylique sont par exemple obtenus à partir d'acide glyoxylique et d'une mono ou polyamine organique.

Cette amine est de préférence choisie parmi la méthylamine, l'éthylamine, la propylamine, l'isopropylamine, la butylamine, l'hexylamine, la monoéthanolamine, la monopropanolamine, la propane-1,2,3-triamine et la diaminoacétone.

On peut en particulier citer le Nbétahydroxyéthylamide de l'acide glyoxylique et le Ngammahydroxypropylamide de l'acide glyoxylique leurs (thio)acétals, hémi(thio)acétals, hydrates.

Les (thio)acétals et hémi(thio)acétals d'acide glyoxylique peuvent par exemple être obtenus à partir de la réaction d'alcools de thiols pour les thioacétals ou hémithioacétals sur des formes bloquées d'acide glyoxylique puis hydrolyse. Les alcools peuvent être les mêmes que ceux cités pour les esters. Les thiols peuvent être des équivalents (appelés mono ou polythiols) aux mono ou polyalcools cités supra à ceci près que la ou les fonctions hydroxy desdits mono ou polyalcools sont remplacées par une ou des fonctions thiols SH des mono ou polythiols. Les acétals ou thioacétals peuvent également être des (thio)acétals cycliques.

Selon un mode de réalisation particulièrement préféré, le composé de formule (I) est l'acide glyoxylique sous forme hydrate.

Selon un mode de réalisation la composition utilisée dans l'invention comprend de 0,5 à 15% d'un ou de plusieurs dérivés dicarbonylés répondant à la formule (I) et/ou d'un de leurs dérivés et/ou de leurs formes hydrate et/ou leurs sels, de préférence de 3 à 15%, préférentiellement de 5 à 10 % en poids du poids total de la composition.

Selon l'invention, la composition comprend un ou plusieurs dérivés aminogènes répondant à la formule **(II)** décrite précédemment.

Selon un mode de réalisation particulier de l'invention les composés de formule **(II)** sont tels que **R₁**, **R₂**, **R₃**, **R₄** représentent, indépendamment les uns de autres :
(i) un atome d'hydrogène, (ii) un radical alkyle ou alcényle en C₁-C₅, linéaire ou ramifié, cyclique ou acyclique, (iii) un radical alcoxy en C₁-C₅, (iv) un radical (hétéro)aryle comprenant de 5 à 18 chainons, de préférence aryle en C₆-C₁₈, (v) un radical (hétéro)cyclique ayant de 5 à 8 chaînons ; ces radicaux (ii) à (v) étant éventuellement substitués notamment par un ou plusieurs radicaux choisis parmi les radicaux : hydroxyle, (C₁-C₄)alkyle, (di)(C₁-C₆)(alkyl)amino tel que diméthylamino, carboxyle, halogène, aryle en C₆-C₁₈, carboxamide et N-méthylcarboxamide ;
étant entendu que
- lorsque R₁, R₂ et R₃ représentent un atome d'hydrogène, R₄ peut désigner un radical carboxamide, méthoxy, éthoxy, 1,2,4-triazolyle, cyclopentyle, (C₁-C₆)alkylcarbonyle tel que acétyle, (C₁-C₆)alcoxycarbonyle tel que méthoxycarbonyle ou éthoxycarbonyle,
- C(O)-C(H)=C(H)-C(O)-OH, phényle éventuellement substitué par un atome de chlore ou un radical hydroxyle, benzyle ou 2,5-dioxo-4-imidazolidinyle ;
- lorsque R₁ et R₃ représentent un atome d'hydrogène, R₂ peut représenter un atome d'hydrogène, un radical méthyle ou éthyle et R₄ un radical acétyle ;
- lorsque R₁=R₂=H, R₃ et R₄ peuvent former, avec l'atome d'azote qui les porte, un cycle pipéridino, 3-méthylpyrazole, 3,5-diméthylpyrazole ou maléimide ;
- R₁ et R₂ ainsi que R₃ et R₄ peuvent former, avec l'atome d'azote qui les porte, un cycle imidazole ;
- **R₁** et **R₃** ou **R₂** et **R₄** pouvant constituer ensemble un radical choisi parmi les radicaux divalents suivants : -CH₂-CH₂-, -CH=CH-, -CH₂-C(O)-, -C(O)-N(H)-, -CH=N-, -C(O)-C(O)-, -CH(OH)-CH(OH)-, -[HO(O)C]CH-CH-, -CH(OH)-C(O)-, -CH₂-CH₂-CH₂-, -CH₂-N(H)-C(O)-, -CH=C(CH₃)-C(O)-, -N(H)-C(O)-N(H)-, -CH₂-CH₂-C(O)-, -CH₂-N(CH₃)-CH₂-, -N(H)-CH₂-N(H)-, -C(O)-CH(CH₃)-CH₂-, -C(O)-CH₂-C(O)-, -C(O)-N(H)-C(O)-, -C(O)-CH[C(O)OH)-CH₂-, -C(O)-CH=C[C(O)OH)]-, -C(O)-CH=C(CH₃)-, -C(O)-C(NH₂)=CH-, -C(O)-C(CH₃)=N-, -C(O)-CH=CH-, -C(O)-CH=N- et -C(O)-N=CH-
- Y est tel que défini précédemment, de préférence Y = O.

De préférence, **R₁**, **R₂**, **R₃**, **R₄**, représentent, indépendamment les uns des autres, un atome ou groupe choisi parmi i) hydrogène, ii) alkyle en C₁-C₃, éventuellement substitué, de préférence par un radical hydroxyle.

Selon une variante, **R₁** et **R₃** ou **R₂** et **R₄** constituent ensemble une chaîne alkylène en C₁-C₃, saturée ou insaturée pouvant éventuellement être substituée par un groupe hydroxyle. Ils forment alors un cycle avec le groupement -N-C(Y)-N-.

De préférence, les dérivés aminogènes répondant à la formule **(II)** sont choisis parmi les dérivés aminogènes répondant à la formule **(II)** dans laquelle Y représente un atome ou groupe choisi parmi l'oxygène et NR.

Selon un mode de réalisation, Y représente un groupe -NH.

De préférence, lorsque R' représente un atome d'hydrogène, R₁ représente un atome d'hydrogène ou un groupe -(CH₂)₃-CH(NH₂)-C(O)-OH.

Lorsque Y représente N-R', le dérivé aminogène est de préférence choisi parmi la guanidine, le carbonate de guanidine, l'arginine, le chlorhydrate d'arginine.

Selon un autre mode de réalisation particulier, Y représente un atome d'oxygène.

Lorsque Y représente un atome d'oxygène, le dérivé aminogène est de préférence choisi parmi l'urée et les dérivés d'urée.

Par dérivé d'urée, on entend tout composé autre que l'urée (NH₂)₂C(O) lui-même, comprenant dans sa formule chimique un groupe carbonyle simplement lié à deux atomes d'azote, c'est-à-dire un motif

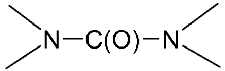

De préférence, l'urée ou le ou les dérivés d'urée sont choisis parmi les composés de formule (a) ou (b), leurs sels, leurs tautomères ou leurs hydrates :

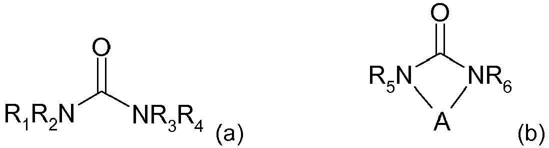

Formule (a) et (b) dans lesquelles :
- **R₁**, **R₂**, **R₃**, **R₄** représentent, indépendamment les uns des autres :
   (i) un atome d'hydrogène ou
   (ii) un radical alkyle ou alcényle en C₁-C₅, linéaire ou ramifié, cyclique ou acyclique un radical alcoxy en C₁-C₅, iii) un radical aryle en C₆-C₁₈, iv) un radical hétérocyclique ayant de 5 à 8 chaînons ; ces radicaux étant éventuellement substitués par un ou plusieurs radicaux choisis parmi les radicaux : hydroxyle, (C₁-C₄)alkyle, (di)(C₁-C₄)(alkyl)amino tel que diméthylamino, carboxyle, halogène, aryle C₆-C₁₈, carboxamide et N-méthylcarboxamide ;
   étant entendu que
- lorsque **R₁**, **R₂** et **R₃** représentent un atome d'hydrogène, R₄ peut désigner un radical carboxamide, méthoxy, éthoxy, 1,2,4-triazolyle, cyclopentyle, (C₁-C₆)alkylcarbonyle tel que acéthyl, (C₁-C₆)alcoxycarbonyle tel que méthoxycarbonyle ou éthoxycarbonyle,
- C(O)-CH=CH-C(O)-OH, phényle éventuellement sustitué par un atome de chlore ou un radical hydroxyle, benzyle ou 2,5-dioxo-4-imidazolidinyle ;
- lorsque R₁ et R₃ représentent un atome d'hydrogène, R₂ peut représenter un atome d'hydrogène, un radical méthyle ou éthyle et R₄ un radical acétyle ;
- lorsque R₁=R₂=H, R₃ et R₄ peuvent former, avec l'atome d'azote qui les porte, un cycle pipéridine, 3-méthylpyrazole, 3,5-diméthylpyrazole ou maléimide ;
- R₁ et R₂ ainsi que R₃ et R₄ peuvent former, avec l'atome d'azote qui les porte, un cycle imidazole ;
- R₅ et R₆ représentent, indépendamment l'un de l'autre,
   (iii) un atome d'hydrogène ou
   (iv) un radical alkyle, acyle, ou alcényle inférieur en C₁-C₅, linéaire ou ramifié, cyclique ou acyclique un radical alcoxy en C₁-C₅, un radical aryle en C₆-C₁₈, un radical hétérocyclique ayant de 5 à 8 chaînons ; ces radicaux étant éventuellement substitués par un radical choisi parmi les radicaux: hydroxyle, amino, diméthylamino, carboxyle, halogène, aryle C₆-C₁₈, carboxamide et N-méthylcarboxamide ;
- **A** est un radical divalent choisi parmi les radicaux divalents suivants : -CH₂-CH₂-, -CH=CH-, -CH₂-C(O)-, -C(O)-N(H)-, -CH=N-, -C(O)-C(O)-, -CH(OH)-CH(OH)-, -[HO(O)C]CH-CH-, -CH(OH)-C(O)-, -CH₂-CH₂-CH₂-, -CH₂-N(H)-C(O)-, -CH=C(CH₃)-C(O)-, -N(H)-C(O)-N(H)-, -CH₂-CH₂-C(O)-, -CH₂-N(CH₃)-CH₂-, -N(H)-CH₂-N(H)-, -C(O)-CH(CH₃)-CH₂-, -C(O)-CH₂-C(O)-, -C(O)-N(H)-C(O)-, -C(O)-CH[C(O)OH]-CH₂-, -C(O)-CH=C[C(O)OH]-, -C(O)-CH=C(CH₃)-, -C(O)-C(NH₂)=CH-, -C(O)-C(CH₃)=N-, -C(O)-CH=CH-, -C(O)-CH=N- et -C(O)-N=CH-.

Parmi les composés de formule (a) particulièrement préférés selon l'invention, on peut citer :
- l'urée
- la méthylurée
- l'éthylurée
- la propylurée
- la n-butylurée
- la sec-butylurée
- l'isobutylurée
- la tert-butylurée
- la cyclopentylurée
- l'éthoxyurée
- l'hydroxyéthylurée
- la N-(2-hydroxypropyl)urée
- la N-(3-hydroxypropyl)urée
- la N-(2-diméthylaminopropyl)urée
- la N-(3-diméthylaminopropyl)urée
- la 1-(3-hydroxyphényl)urée
- la benzylurée
- la N-carbamoyl maléamide
- l'acide N-carbamoyl maléamique
- le pipéridinocarboxamide
- la 1,2,4-triazol-4-yl-urée
- l'acide hydantoïque
- l'hydanthoïne
- l'allophanate de méthyle
- l'allophanate d'éthyle
- l'acétylurée
- l'hydroxyéthylèneurée
- la 2-(hydroxyéthyl)éthylèneurée
- la diallylurée
- la chloroéthylurée
- la N,N-diméthylurée
- la N,N-diéthylurée
- la N,N-dipropylurée
- la cyclopentyl-1-méthylurée
- la 1,3-diméthylurée
- la 1,3-diéthylurée
- la 1,3-bis(2-hydroxyéthyl)urée
- la 1,3-bis(2-hydroxypropyl)urée
- la 1,3-bis(3-hydroxypropyl)urée
- la 1,3-dipropylurée
- l'éthyl-3-propylurée
- la sec-butyl-3-méthylurée
- l'isobutyl-3-méthylurée
- la cyclopentyl-3-méthylurée
- la N-acétyl-N'-méthylurée
- la triméthylurée
- la butyl-3,3-diméthylurée
- la tétraméthylurée et
- la benzylurée.

Parmi les composés de formule (b) particulièrement préférés selon l'invention, on peut citer :
- l'acide parabanique
- le 1,2-dihydro-3-H-1,2,4-triazol-2-one
- l'acide barbiturique
- l'uracile
- le 1-méthyl uracile
- le 3-méthyl uracile
- le 5-méthyl uracile
- le 1,3-diméthyl uracile
- le 5-aza uracile
- le 6-aza uracile
- le 5-fluoro uracile
- le 6-fluoro uracile
- le 1,3-diméthyl-5-fluoro uracile
- le 5-amino uracile
- le 6-amino uracile
- le 6-amino-1-méthyl uracile
- le 6-amino-1,3-diméthyl uracile
- le 4-chloro uracile
- le 5-chloro uracile
- le 5,6-dihydro uracile
- le 5,6-dihydro-5-méthyle uracile
- la 2-imidazolidone
- la 1-méthyl-2-imidazolidinone
- la 1,3-diméthyl-2-imidazolidinone
- la 4,5-dihydroxy-imidazolidin-2-one
- la 1-(2-hydroxyéthyl)-2-imidazolidinone
- la 1-(2-hydroxypropyl)-2-imidazolidinone
- la 1-(3-hydroxypropyl)-2-imidazolidinone
- la 4,5-dihydroxy-1,3-diméthyl-imidazolidin-2-one
- la 1,3-bis(2-hydroxyéthyl)-2-imidazolidinone
- l'acide 2-imidazolidone-4-carboxylique
- le 1-(2-aminoéthyl)-2-imidazole
- la 4-méthyl-1,2,4-triazoline-3,5-dione
- la 2,4-dihydroxy-6-méthylpyrimidine
- la 1-amino-4,5-dihydro-1H-tétrazol-5-one
- l'hydantoïne
- la 1-méthylhydantoïne
- la 5-méthylhydantoïne
- la 5,5-diméthylhydantoïne
- la 5-éthylhydantoïne
- la 5-n-propylhydantoïne
- la 5-éthyl-5-méthylhydantoïne
- la 5-hydroxy-5-méthylhydantoïne
- la 5-hydroxyméthylhydantoïne
- la 1-allylhydantoïne
- la 1-aminohydantoïne
- l'acide hydantoïne 5-acétique
- le 4-amino-1,2,4-triazolone-3,5-dione
- l'hexahydro-1,2,4,5-tétrazine-3,6-dione
- le 5-méthyl-1,3,5-triazinon-2-one
- la 1-méthyl-tétrahydro-pyrimidin-2-one
- la 2,4-dioxohexahydro-1,3,5-triazine
- l'urazole
- le 4-méthylurazole
- l'acide orotique
- l'acide dihydroxyorotique
- la 2,4,5-trihydroxypyrimidine
- la 2-hydroxy-4-méthylpyrimidine
- la 4,5-diamino-2,6-dihydroxypyrimidine
- l'acide barbiturique
- l'acide 1,3-diméthylbarbiturique
- l'acide cyanurique
- la 1-méthyl-hexahydropyrimidine-2,4-dione
- la 1,3-diméthyl-3,4,5,6-tétrahydro-2-1H-pyrimidinone
- la 5-(hydroxyméthyl-2,4-(1H,3H)-pyrimidinedione
- l'acide 2,4-dihydroxypyrimidine-5-carboxylique
- la 6-azathymine
- la 5-méthyl-1,3,5-triazinan-2-one
- l'acide N-carbamoylmaléamique et
- l'alloxane monohydraté.

De préférence le composé aminogène est choisi parmi l'arginine, la guanidine, l'urée, l'hydroxyéthylurée, leurs sels, leurs hydrates ou leurs mélanges. Encore plus préférentiellement le composé aminogène est l'urée.

Selon un mode de réalisation la composition utilisée dans l'invention comprend de 0,01 à 15% en poids de dérivés aminogènes répondant à la formule (II), leurs sels ou leurs hydrates, de préférence de 0,1 à 10% en poids, mieux de 0,2 à 5 % en poids par rapport au poids total de la composition.

Le ratio pondéral dérivés dicarbonylés répondant à la formule (I) et/ou d'un de leurs dérivés et/ou de leurs formes hydrate et/ou leurs sels/dérivés aminogènes répondant à la formule (II) ou de leurs formes hydrate et/ou leurs sels peut de préférence varier de 0,1 à 100, mieux de 1 à 20.

Selon un mode de réalisation particulier, la composition comprend au moins un tensioactif amphotère ou zwittérionique.

En particulier, le ou les tensioactifs amphotères ou zwittérioniques, de préférence non siliconés, utilisables dans la présente invention, peuvent être notamment des dérivés d'amines aliphatiques secondaire ou tertiaire, éventuellement quaternisées, dans lesquels le groupe aliphatique est une chaîne linéaire ou ramifiée comportant de 8 à 22 atomes de carbone, lesdits dérivés d'amines contenant au moins un groupe anionique tel que, par exemple, un groupe carboxylate, sulfonate, sulfate, phosphate ou phosphonate.

On peut citer en particulier les alkyl(C₈-C₂₀)bétaïnes, les alkyl(C₈-C₂₀) sulfobétaïnes, les alkyl(C₈-C₂₀)amidoalkyl(C₃-C₈)bétaïnes et les alkyl(C₈-C₂₀)-amidalkyl(C₆-C₈)sulfobétaïnes.

Parmi les dérivés d'amines aliphatiques secondaires ou tertiaires, éventuellement quaternisées utilisables, tels que définis ci-dessus, on peut également citer les composés de structures respectives **(B1)** et **(B2)** suivantes :

Rₐ-C(O)-NH-CH₂-CH₂-N⁺(R_{b})(R_{c})-CH₂C(O)O⁻, M⁺, X⁻ **(B1)**

Formule dans laquelle :
▪ Rₐ représente un groupe alkyle ou alcényle en C₁₀-C₃₀ dérivé d'un acide RₐCOOH, de préférence présent dans l'huile de coprah hydrolysée, un groupe heptyle, nonyle ou undécyle ;
▪ R_{b} représente un groupe bêta-hydroxyéthyle ; et
▪ R_{c} représente un groupe carboxyméthyle ;
▪ M⁺ représente un contre ion cationique issu d'un métal alcalin, alcalinoterreux, tel que le sodium, un ion ammonium ou un ion issu d'une amine organique, et
▪ X⁻ représente un contre ion anionique organique ou inorganique, tel que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate ; ou alors M⁺ et X⁻ sont absents ;

R_{a'}-C(O)-NH-CH₂-CH₂-N(B)(B') **(B2)**

Formule dans laquelle :
▪ B représente le groupe -CH₂-CH₂-O-X' ;
▪ B' représente le groupe -(CH₂)_{z}Y', avec z = 1 ou 2 ;
▪ X' représente le groupe -CH₂-C(O)OH, -CH₂-C(O)OZ', -CH₂-CH₂-C(O)OH, -CH₂-CH₂-C(O)OZ', ou un atome d'hydrogène ;
▪ Y' représente le groupe -C(O)OH, -C(O)OZ', -CH₂-CH(OH)-SO₃H ou le groupe -CH₂-CH(OH)-SO₃-Z' ;
▪ Z' représente un contre ion cationique issu d'un métal alcalin ou alcalinoterreux, tel que le sodium, un ion ammonium ou un ion issu d'une amine organique ;
▪ R_{a'} représente un groupe alkyle ou alcényle en C₁₀-C₃₀ d'un acide R_{a'}-C(O)OH de préférence présent dans l'huile de coprah ou dans l'huile de lin hydrolysée, un groupe alkyle, notamment en C₁₇ et sa forme iso, un groupe en C₁₇ in saturé.

Les composés de ce type sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré

On peut aussi utiliser des composés de formule (B'2) ;

**Ra"-NH-CH(Y")-(CH2)n-C(O)-NH-(CH2)n'-N(Rd)(Re)** **(B'2)**

Formule dans laquelle :
▪ Y" représente le groupe -C(O)OH, -C(O)OZ", -CH2-CH(OH)-SO3H ou le groupe -CH2-CH(OH)-SO3-Z" ;
▪ Rd et Re, indépendamment l'un de l'autre, représentent un radical alkyle ou hydroxyalkyle en C1_C4
▪ Z" représente un contre ion cationique issu d'un métal alcalin ou alcalinoterreux, tel que le sodium, un ion ammonium ou un ion issu d'une amine organique ;
▪ Ra" représente un groupe alkyle ou alcényle en C10-C30 d'un acide Ra"-C(O)OH de préférence présent dans l'huile de coprah ou dans l'huile de lin hydrolysée.
▪ n et n', indépendamment l'un de l'autre, désignent un nombre entier allant de 1 à 3.

Parmi les composés de formule (B'2) on peut citer le composé classé dans le dictionnaire CTFA sous la dénomination sodium diethylaminopropyl cocoaspartamide et commercialisé par la société CHIMEX sous l'appellation CHIMEXANE HB.

Conformément à un mode de réalisation particulier de l'invention, la teneur en tensioactif(s) amphotère(s) ou zwittérionique(s), lorsqu'il(s) est(sont) présent(s), varie de 0,05 à 30 % en poids, de préférence de 0,5 à 10 % en poids, et de manière plus préférée de 0,1 à 5 % en poids, par rapport au poids total de la composition.

La composition utilisée dans l'invention peut également comprendre un polymère cellulosique.

Par polymère « *cellulosique* », on entend selon l'invention tout composé polysaccharidique possédant dans sa structure des enchaînements de résidus glucose unis par des liaisons β-1,4 ; outre les celluloses non substituées, les dérivés de celluloses peuvent être anioniques, cationiques, amphotères ou non-ioniques. Ainsi, les polymères cellulosiques de l'invention peuvent être choisis parmi les celluloses non substituées y compris sous une forme microcristalline et les éthers de cellulose. Parmi ces polymères cellulosiques, on distingue les éthers de celluloses, les esters de celluloses et les esters éthers de celluloses. Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulfates d'éthylcellulose.

Les compositions utilisée dans l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.

Ces compositions peuvent être conditionnées dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

Les compositions utilisée dans l'invention peuvent être aqueuses ou anhydres. Elles sont de préférence aqueuses et comprennent alors de l'eau à une concentration allant de 5 à 98%, mieux de 5 à 90%, encore mieux de 10 à 90 % en poids par rapport au poids total de la composition.

La composition peut notamment comprendre un ou plusieurs solvants organiques notamment hydrosolubles tels que les alcools en C₆-C₇; on peut notamment citer les monoalcools aliphatiques en C₁-C₇ ou aromatiques en C₆-C₇, les polyols et les éthers de polyols en C₃-C₇, qui peuvent être employés seuls ou en mélange avec de l'eau.

La composition utilisée dans l'invention peut en outre comprendre au moins un ingrédient cosmétique usuel, notamment choisi parmi les propulseurs; les huiles; les corps gras solides et notamment les esters en C₈-C₄₀, les acides en C₈-C₄₀; les alcools en C₈-C₄₀, les tensioactifs autres que ceux décrits précédemment, les filtres solaires; les agents hydratants; les agents antipelliculaires; les agents antioxydants; les agents chélatants; les agents nacrants et opacifiants; les agents plastifiants ou de coalescence; les charges; les silicones et en particulier les polydiméthylsiloxanes; les épaississants ou gélifiants , polymériques ou non;autres que les polymères cellulosiques déjà mentionnés ; les émulsionnants; les polymères notamment conditionneurs ou coiffants; les parfums; les agents d'alcalinisation tels que la soude ou d'acidification; les silanes ; les agents de réticulation. La composition peut bien évidemment comprendre plusieurs ingrédients cosmétiques figurant dans la liste ci-dessus.

Selon leur nature et la destination de la composition, les ingrédients cosmétiques usuels peuvent être présents en des quantités usuelles, aisément déterminables par l'homme du métier, et qui peuvent être comprises, pour chaque ingrédient, entre 0,01 à 80% en poids. L'homme de métier veillera à choisir les ingrédients entrant dans la composition, ainsi que leurs quantités, de manière à ce qu'ils ne nuisent pas aux propriétés des compositions de la présente invention.

Le pH de la composition est de préférence inférieur à 4, et varie préférentiellement de 1 à 3, mieux de 1,5 à 3, encore mieux de 1,7 à 3.

Il peut être ajusté à la valeur désirée au moyen d'agents alcalinisants et/ou acidifiants habituellement utilisés pour le traitement des fibres kératiniques.

L'agent alcalinisant peut être choisi parmi les agents alcalins minéraux ou organiques ou hybrides ou leurs mélanges.

Le ou les agents alcalins minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.

Le ou les agents alcalins organiques sont de préférence choisis parmi les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

A titre de composés hybrides on peut mentionner les sels des amines citées précédemment avec des acides comme l'acide carbonique, l'acide chlorhydrique.
Le ou les agents alcalins organiques sont par exemple choisis parmi les dérivés aminés tels que alcanolamines, les éthylènediamines oxyéthylénées et/ou oxypropylénées, les acides aminés des amines telles que le 1,3 diaminopropane, le 1,3 diamino 2 propanol, la spermine, la spermidine.

Par alcanolamine on entend une amine organique comprenant une fonction amine primaire, secondaire ou tertiaire, et un ou plusieurs groupements alkyle, linéaires ou ramifiés, en C₁-C₈ porteurs d'un ou plusieurs radicaux hydroxyle.

Convient en particulier à la réalisation de l'invention l'hydroxyde de sodium.

L'agent acidifiant peut être choisi parmi les acides minéraux ou organiques, comme par exemple l'acide chlorhydrique, l'acide phosphorique, l'acide lactique.

La composition utilisée dans l'invention se présente de préférence sous forme de gels de coiffage ou de soin, de lotions ou crèmes de soin, de conditionneurs, de masques, de sérums.

La composition utilisée dans l'invention peut être obtenue par mélange de plusieurs compositions.

Le procédé de l'invention comprend l'application de la composition décrite précédemment suivi d'une étape de lissage au fer des cheveux de préférence à une température d'au moins 150°C. Le lissage au fer est connu de l'état de la technique. Il consiste à lisser les cheveux avec une pince plate chauffante, généralement métallique. Les fers de lissage sont généralement utilisés à une température variant de 150 à 250°C.

Le procédé de l'invention peut comprendre d'autres étapes intermédiaires visant à améliorer le lissage des cheveux.

Selon un mode de réalisation particulier, le procédé de l'invention comprend l'application de la composition sur cheveux secs, un temps de contact de la composition sur les cheveux allant de 10 à 60 minutes, de préférences de 20 à 40 minutes. Après ce temps de pose, on effectue un lissage à la brosse et au sèche-cheveux (brushing). On lisse ensuite les cheveux au fer à lisser à une température allant de 150 à 250°C, de préférence de 210 à 230°C.

Le procédé de l'invention peut comprendre l'application d'autres agents capillaires en pré- ou post traitement. Notamment, il peut comprendre l'application d'un soin de conditionnement en post traitement.

Selon un autre mode de réalisation, le procédé de lissage des cheveux comprend une étape de lavage des cheveux puis de séchage au sèche-cheveux avant application de la composition de l'invention. Selon ce mode de réalisation particulier, on retrouve ensuite les étapes décrites ci-dessus telles que le temps de contact de la composition, le lissage au fer à lisser, l'application d'un agent de conditionnement et le rinçage, toutes ces étapes pouvant être mises en oeuvre indépendamment l'une de l'autre, un brushing pouvant s'intercaler entre le contact de la composition selon l'invention et le lissage au fer. Selon un mode de réalisation particulier, le lissage au fer à lisser s'effectue en plusieurs passages sur les cheveux, en général 8 à 10 passages.

Le procédé de la présente invention est mis en oeuvre, de préférence, sans une étape de déformation permanente à pH basique ni à base de réducteur.

### Exemples

On réalise les compositions suivantes, les compositions 1, 2 et 3 étant des compositions conformes à l'invention et les compositions C1 à C6 étant des exemples comparatifs.

### Exemples de réalisation :

| | 1 | C1 | C2 | C3 | 2 | 3 | C4 | C5 | C6 |
|---|---|---|---|---|---|---|---|---|---|
| Acide glyoxylique | 5g | 5g | - | - | 5 g | - | - | - | - |
| Acide pyruvique | - | - | - | - | - | 8g | 8g | - | - |
| Urée | 0,4g | - | 0,4g | 0,4g | - | - | - | - | - |
| Hydroxyéthylurée | - | - | - | - | - | 3g | | 3g | 3g |
| Carbonate de guanidine | - | - | - | - | 0,5g | - | - | - | - |
| | | | | | | | | | |
| Soude/ Acide chlorhydrique | QS | QS | - | QS | QS | QS | QS | - | QS |
| | pH | pH | | pH | pH | pH | pH | | pH |
| | 2,2 | 2,2 | | 2,2 | 2,2 | 2,2 | 2,2 | | 2,2 |
| Eau | QS | QS | QS | QS | QS | QS | QS | QS | QS |
| | 100g | 100g | 100g | 100g | 100g | 100g | 100g | 100g | 100g |

Les compositions sont appliquées sur des cheveux bouclés qui peuvent être naturels ou colorés, ou sensibilisés par une étape préalable de décoloration à raison de 1g pour 2g de cheveu. Après 15 minutes, les cheveux sont rincés, séchés au sèche-cheveu (brushing) puis lissés par le passage de pinces plates portées à 210°C. Ils sont ultérieurement soumis à des shampooings pour examiner la rémanence des effets de lissage et de modification des propriétés mécaniques et cosmétiques des fibres.

Les compositions C2 et C3 ne permettent pas d'obtenir de lissage permanent. Les compositions 1 et 2 suivant l'invention permettent d'obtenir des propriétés de lissage supérieures à celles obtenues avec la composition C1 en termes de détente de boucle, de protection de la couleur naturelle comme artificielle, de résistance des fibres aux contraintes mécaniques (traction, frottement, torsion), de brillance, de toucher et de visuel lisse.

La composition 3 suivant l'invention permet d'obtenir des propriétés de lissage supérieures à celles obtenues avec la composition C4, C5 ou C6.

## Revendications

1. Procédé de lissage des fibres kératiniques, notamment des cheveux, qui comprend l'application sur les cheveux d'une composition cosmétique comprenant
- un ou plusieurs composés dicarbonylés répondant à la formule (I) suivante et/ou leurs dérivés et/ou leurs hydrates et/ou leurs sels, les dérivés étant choisis parmi les esters du ou des groupes carboxy, les amides du ou des groupes carboxy, les (thio)acétals et hémi(thio)acétals de la ou des fonctions carbonyles des composés de formule **(I)** sous forme libre ou éventuellement sous forme de sels ou d'hydrates, de préférence sous forme libre ou d'hydrates : 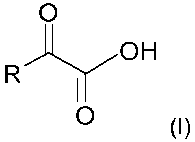 formule (I) dans laquelle
R représente un atome ou groupe choisi parmi i) hydrogène, ii) carboxy -C(O)OH, iii) alkyle linéaire ou ramifié en C₁-C₆ éventuellement substitué de préférence par au moins un radical hydroxy -OH, carboxy -C(O)-OH ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyl et vii) un radical imidazolylméthyle et ses tautomères tel que 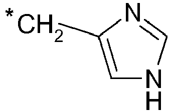 avec * représentant la partie reliée au reste de la molécule,
- un ou plusieurs dérivés aminogènes choisis parmi l'urée ou les dérivés d'urée, leurs sels ou leurs hydrates, les dérivés d'urée étant choisis parmi des composés, autres que l'urée, comprenant dans leur formule chimique un groupe carbonyle simplement lié à deux atomes d'azote, c'est-à-dire un motif 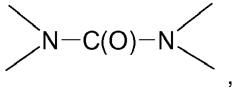 suivi d'une étape de lissage au moyen d'un fer de lissage à une température d'au moins 150°C, de préférence allant de 150 à 250°C.

2. Procédé selon la revendication 1, dans lequel R représente i) un atome d'hydrogène ou ii) un groupe alkyle linéaire ou ramifié en C₁-C₆ éventuellement substitué par un groupe carboxy.

3. Procédé selon l'une quelconque des revendications précédentes dans lequel le ou les composés dicarbonylés répondant à la formule (I) et/ou leurs dérivés et/ou leurs hydrates et/ou leurs sels sont choisis parmi l'acide glyoxylique, l'acide pyruvique, l'un de leurs dérivés, leurs sels et leurs hydrates, de préférence parmi l'acide glyoxylique, l'un de ses dérivés et les formes hydrate de ces composés.

4. Procédé selon l'une quelconque des revendications précédentes, dans lequel le ou les dérivés dicarbonylés répondant à la formule (I) et/ou leurs dérivés sont choisis parmi les esters d'acide glyoxylique, les amides d'acide glyoxylique, les (thio)acétals et hémi(thio)acétals d'acide glyoxylique, les (thio)acétals et hémi(thio)acétals d'ester d'acide glyoxylique.

5. Procédé selon la revendication 3, dans lequel l'acide glyoxylique se présente sous sa forme hydrate.

6. Procédé selon l'une quelconque des revendications 1 à 5, dans lequel la composition comprend de 0,5 à 15% en poids d'un ou de plusieurs dérivés dicarbonylés répondant à la formule (I) et/ou leurs dérivés et/ou leurs hydrates et/ou leurs sels, de préférence de 3 à 15%, préférentiellement de 5 à 10 % en poids du poids total de la composition.

7. Procédé selon l'une quelconque des revendications précédentes dans lequel l'urée ou les dérivés d'urée sont choisis parmi les composés de formule (a) ou (b), leurs sels, tautomères, ou leurs hydrates : 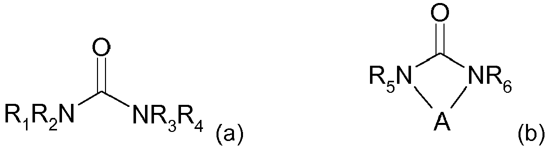 Formule (a) et (b) dans lesquelles :
- **R₁**, **R₂**, **R₃**, **R₄** représentent, indépendamment les uns des autres :
(i) un atome d'hydrogène ou
(ii) un radical alkyle ou alcényle en C₁-C₅, linéaire ou ramifié, cyclique ou acyclique un radical alcoxy en C₁-C₅, iii) un radical aryle en C₆-C₁₈, iv) un radical hétérocyclique ayant de 5 à 8 chaînons ; ces radicaux étant éventuellement substitués par un ou plusieurs radicaux choisis parmi les radicaux : hydroxyle, (C₁-C₄)alkyle, (di)(C₁-C₄)(alkyl)amino tel que diméthylamino, carboxyle, halogène, aryle C₆-C₁₈, carboxamide et N-méthylcarboxamide ;
étant entendu que
- lorsque **R₁**, **R₂** et **R₃** représentent un atome d'hydrogène, R₄ peut désigner un radical carboxamide, méthoxy, éthoxy, 1,2,4-triazolyle, cyclopentyle, (C₁-C₆)alkylcarbonyle tel que acéthyl, (C₁-C₆)alcoxycarbonyle tel que méthoxycarbonyle ou éthoxycarbonyle, -C(O)-CH=CH-C(O)-OH, phényle éventuellement sustitué par un atome de chlore ou un radical hydroxyle, benzyle ou 2,5-dioxo-4-imidazolidinyle ;
- lorsque **R₁** et **R₃** représentent un atome d'hydrogène, **R₂** peut représenter un atome d'hydrogène, un radical méthyle ou éthyle et R₄ un radical acétyle ;
- lorsque **R₁**=**R₂**=H, **R₃** et **R₄** peuvent former, avec l'atome d'azote qui les porte, un cycle pipéridine, 3-méthylpyrazole, 3,5-diméthylpyrazole ou maléimide ;
- **R₁** et **R₂** ainsi que **R₃** et **R₄** peuvent former, avec l'atome d'azote qui les porte, un cycle imidazole ;
- **R₅** et **R₆** représentent, indépendamment l'un de l'autre,
(iii) un atome d'hydrogène ou
(iv) un radical alkyle, acyle, ou alcényle inférieur en C₁-C₅, linéaire ou ramifié, cyclique ou acyclique un radical alcoxy en C₁-C₅, un radical aryle en C₆-C₁₈, un radical hétérocyclique ayant de 5 à 8 chaînons ; ces radicaux étant éventuellement substitués par un radical choisi parmi les radicaux: hydroxyle, amino, diméthylamino, carboxyle, halogène, aryle C₆-C₁₈, carboxamide et N-méthylcarboxamide ;
- **A** est un radical divalent choisi parmi les radicaux divalents suivants : -CH₂-CH₂-, -CH=CH-, -CH₂-C(O)-, -C(O)-N(H)-, -CH=N-, -C(O)-C(O)-, -CH(OH)-CH(OH)-, -[HO(O)C]CH-CH-, -CH(OH)-C(O)-, -CH₂-CH₂-CH₂-, -CH₂-N(H)-C(O)-, -CH=C(CH₃)-C(O)-, -N(H)-C(O)-N(H)-, -CH₂-CH₂-C(O)-, -CH₂-N(CH₃)-CH₂-, -N(H)-CH₂-N(H)-, -C(O)-CH(CH₃)-CH₂-, -C(O)-CH₂-C(O)-, -C(O)-N(H)-C(O)-, -C(O)-CH[C(O)OH]-CH₂-, -C(O)-CH=C[C(O)OH]-, -C(O)-CH=C(CH₃)-, -C(O)-C(NH₂)=CH-, -C(O)-C(CH₃)=N-, -C(O)-CH=CH-, -C(O)-CH=N- et -C(O)-N=CH-.

8. Procédé selon la revendication précédente dans lequel l'urée ou les dérivés d'urée sont choisis parmi les composés suivants, leurs sels, tautomères, ou leurs hydrates:
- l'urée
- la méthylurée
- l'éthylurée
- la propylurée
- la n-butylurée
- la sec-butylurée
- l'isobutylurée
- la tert-butylurée
- la cyclopentylurée
- l'éthoxyurée
- l'hydroxyéthylurée
- la N-(2-hydroxypropyl)urée
- la N-(3-hydroxypropyl)urée
- la N-(2-diméthylaminopropyl)urée
- la N-(3-diméthylaminopropyl)urée
- la 1-(3-hydroxyphényl)urée
- la benzylurée
- la N-carbamoyl maléamide
- l'acide N-carbamoyl maléamique
- le pipéridinocarboxamide
- la 1,2,4-triazol-4-yl-urée
- l'acide hydantoïque
- l'allophanate de méthyle
- l'allophanate d'éthyle
- l'acétylurée
- l'hydroxyéthylèneurée
- la 2-(hydroxyéthyl)éthylèneurée
- la diallylurée
- la chloroéthylurée
- la N,N-diméthylurée
- la N,N-diéthylurée
- la N,N-dipropylurée
- la cyclopentyl-1-méthylurée
- la 1,3-diméthylurée
- la 1,3-diéthylurée
- la 1,3-bis(2-hydroxyéthyl)urée
- la 1,3-bis(2-hydroxypropyl)urée
- la 1,3-bis(3-hydroxypropyl)urée
- la 1,3-dipropylurée
- l'éthyl-3-propylurée
- la sec-butyl-3-méthylurée
- l'isobutyl-3-méthylurée
- la cyclopentyl-3-méthylurée
- la N-acétyl-N'-méthylurée
- la triméthylurée
- la butyl-3,3-diméthylurée
- la tétraméthylurée et
- la benzylurée.
- l'acide parabanique
- le 1,2-dihydro-3-H-1,2,4-triazol-2-one
- l'uracile
- le 1-méthyl uracile
- le 3-méthyl uracile
- le 5-méthyl uracile
- le 1,3-diméthyl uracile
- le 5-aza uracile
- le 6-aza uracile
- le 5-fluoro uracile
- le 6-fluoro uracile
- le 1,3-diméthyl-5-fluoro uracile
- le 5-amino uracile
- le 6-amino uracile
- le 6-amino-1-méthyl uracile
- le 6-amino-1,3-diméthyl uracile
- le 4-chloro uracile
- le 5-chloro uracile
- le 5,6-dihydro uracile
- le 5,6-dihydro-5-méthyle uracile
- la 2-imidazolidone
- la 1-méthyl-2-imidazolidinone
- la 1,3-diméthyl-2-imidazolidinone
- la 4,5-dihydroxy-imidazolidin-2-one
- la 1-(2-hydroxyéthyl)-2-imidazolidinone
- la 1-(2-hydroxypropyl)-2-imidazolidinone
- la 1-(3-hydroxypropyl)-2-imidazolidinone
- la 4,5-dihydroxy-1,3-diméthyl-imidazolidin-2-one
- la 1,3-bis(2-hydroxyéthyl)-2-imidazolidinone
- l'acide 2-imidazolidone-4-carboxylique
- le 1-(2-aminoéthyl)-2-imidazole
- la 4-méthyl-1,2,4-triazoline-3,5-dione
- la 2,4-dihydroxy-6-méthylpyrimidine
- la 1-amino-4,5-dihydro-1H-tétrazol-5-one
- l'hydantoïne
- la 1-méthylhydantoïne
- la 5-méthylhydantoïne
- la 5,5-diméthylhydantoïne
- la 5-éthylhydantoïne
- la 5-n-propylhydantoïne
- la 5-éthyl-5-méthylhydantoïne
- la 5-hydroxy-5-méthylhydantoïne
- la 5-hydroxyméthylhydantoïne
- la 1-allylhydantoïne
- la 1-aminohydantoïne
- l'acide hydantoïne 5-acétique
- le 4-amino-1,2,4-triazolone-3,5-dione
- l'hexahydro-1,2,4,5-tétrazine-3,6-dione
- le 5-méthyl-1,3,5-triazinon-2-one
- la 1-méthyl-tétrahydro-pyrimidin-2-one
- la 2,4-dioxohexahydro-1,3,5-triazine
- l'urazole
- le 4-méthylurazole
- l'acide orotique
- l'acide dihydroxyorotique
- la 2,4,5-trihydroxypyrimidine
- la 2-hydroxy-4-méthylpyrimidine
- la 4,5-diamino-2,6-dihydroxypyrimidine
- l'acide barbiturique
- l'acide 1,3-diméthylbarbiturique
- l'acide cyanurique
- la 1-méthyl-hexahydropyrimidine-2,4-dione
- la 1,3-diméthyl-3,4,5,6-tétrahydro-2-1H-pyrimidinone
- la 5-(hydroxyméthyl-2,4-(1H,3H)-pyrimidinedione
- l'acide 2,4-dihydroxypyrimidine-5-carboxylique
- la 6-azathymine
- la 5-méthyl-1,3,5-triazinan-2-one
- l'acide N-carbamoylmaléamique et
- l'alloxane monohydraté.

9. Procédé selon l'une quelconque des revendications précédentes dans lequel le ou les dérivés aminogènes répondant à la formule **(II)** sont choisis parmi l'urée, l'hydroxyéthylurée, leurs sels, leurs hydrates et plus particulièrement l'urée ses sels, et ses hydrates.

10. Procédé selon l'une des revendications précédentes, **caractérisé en ce que** le ou les dérivés aminogènes répondant à la formule **(II)**, leurs sels, leurs hydrates sont présents en une teneur en matière active allant de 0,01 à 15% en poids, de préférence de 0,1 à 10% en poids, de préférence allant de 0,2 à 5 % en poids par rapport au poids total de la composition.

11. Procédé selon l'une quelconque des revendications précédentes dans lequel le ratio pondéral dérivés dicarbonylés répondant à la formule **(I)** et/ou d'un de leurs dérivés et/ou de leurs formes hydrate et/ou leurs sels/dérivés aminogènes répondant à la formule **(II)** ou de leurs formes hydrate et/ou leurs sels varie de 0,1 à 100, mieux de 1 à 20.

12. Procédé selon l'une quelconque des revendications précédentes **caractérisé en ce que** la composition est aqueuse et comprend de l'eau à une concentration de préférence allant de 5 à 98%, mieux de 5 à 90%, encore mieux de 10 à 90 % en poids par rapport au poids total de la composition.

13. Procédé selon l'une quelconque des revendications précédentes **caractérisé en ce que** la composition présente un pH inférieur à 4, le pH variant préférentiellement de 1 à 3, mieux de 1,5 à 3, encore mieux de 1,7 à 3.

14. Procédé selon l'une quelconque des revendications précédentes **caractérisé en ce que** la composition résulte du mélange de plusieurs compositions.

15. Procédé selon l'une quelconque des revendications précédentes dans lequel l'application de la composition est suivie d'un temps de contact allant de 10 à 60 minutes.

## Patentansprüche

1. Verfahren zum Glätten von Keratinfasern, insbesondere dem Haar, bei dem man auf das Haar eine kosmetische Zusammensetzung, die
- eine oder mehrere Dicarbonylverbindungen gemäß nachstehender Formel (I) und/oder Derivate davon und/oder Hydrate davon und/oder Salze davon, wobei die Derivate aus Estern der Carboxylgruppe(n), Amiden der Carboxylgruppe(n), (Thio)acetalen und Hemi(thio)acetalen der Carbonylfunktion(en) der Verbindungen der Formel **(I)** in freier Form oder gegebenenfalls in Form von Salzen oder Hydraten, vorzugsweise in freier Form oder in Form von Hydraten, ausgewählt sind: 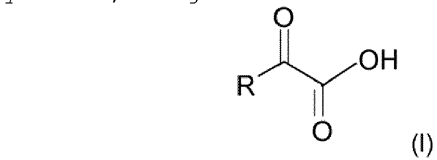 wobei in Formel (I)
R für ein Atom oder eine Gruppe steht, das bzw. die ausgewählt ist aus: i) Wasserstoff, ii) Carboxy -C(O)OH, iii) geradkettigem oder verzweigtem C₁-C₆-Alkyl, das gegebenenfalls substituiert ist, vorzugsweise durch mindestens einen Hydroxylrest -OH, Carboxylrest -C(O)OH oder Halogenrest wie Br; iv) gegebenenfalls substituiertem Phenyl, v) gegebenenfalls substituiertem Benzyl, wobei iv) und v) vorzugsweise gegebenenfalls durch mindestens einen -OH- oder -C(O)OH-Rest substituiert sind; vi) einem Indolylrest und vii) einem Imidazolylmethylrest und Tautomeren davon wie 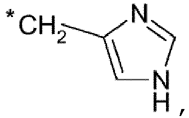 wobei * für den mit dem Rest des Moleküls verknüpften Teil steht,
- ein oder mehrere aminbildende Derivate, die aus Harnstoff oder Harnstoffderivaten, Salzen davon oder Hydraten davon ausgewählt sind, wobei die Harnstoffderivate aus Verbindungen außer Harnstoff, die in ihrer chemischen Formel eine einfach an zwei Kohlenstoffatome gebundene Carbonylgruppe, d. h. eine Einheit 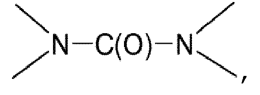 umfassen, ausgewählt sind,
umfasst, aufbringt, worauf ein Glättungsschritt unter Verwendung eines Glätteisens bei einer Temperatur von mindestens 150 °C, vorzugsweise im Bereich von 150 bis 250 °C, folgt.

2. Verfahren nach Anspruch 1, wobei R für i) ein Wasserstoffatom oder ii) eine geradkettige oder verzweigte C₁-C₆-Alkylgruppe, die gegebenenfalls durch eine Carboxygruppe substituiert ist, steht.

3. Verfahren nach einem der vorhergehenden Ansprüche, wobei die Dicarbonylverbindung(en) der Formel (I) und/oder Derivate davon und/oder Hydrate davon und/oder Salze davon aus Glyoxylsäure, Brenztraubensäure, einem ihrer Derivate, ihrer Salze und ihrer Hydrate, vorzugsweise aus Glyoxylsäure, einem ihrer Derivate und den Hydratformen dieser Verbindungen, ausgewählt ist bzw. sind.

4. Verfahren nach einem der vorhergehenden Ansprüche, wobei die Dicarbonylverbindung(en) der Formel (I) und/oder Derivate davon aus Glyoxylsäureestern, Glyoxylsäureamiden, Glyoxylsäure(thio)acetalen und -hemi(thio)acetalen und Glyoxylsäureester(thio)-acetalen und -hemi(thio)acetalen ausgewählt ist bzw. sind.

5. Verfahren nach Anspruch 3, wobei die Glyoxylsäure in ihrer Hydratform vorliegt.

6. Verfahren nach einem der Ansprüche 1 bis 5, wobei die Zusammensetzung 0,5 bis 15 Gew.-% eines oder mehrerer Dicarbonylderivate der Formel (I) Derivate davon und/oder Hydrate davon und/oder Salze davon, vorzugsweise 3 bis 15 Gew.-%, bevorzugt 5 bis 10 Gew.-%, bezogen auf das Gesamtgewicht der Zusammensetzung, umfasst.

7. Verfahren nach einem der vorhergehenden Ansprüche, wobei der Harnstoff bzw. die Harnstoffderivate aus den Verbindungen der Formel (a) oder (b), Salzen davon, Tautomeren oder Hydraten davon ausgewählt ist bzw. sind: 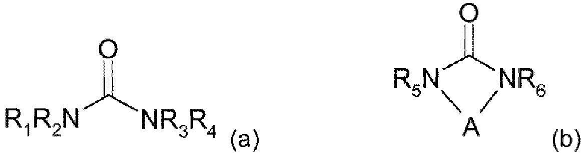 wobei in den Formeln (a) und (b):
- **R₁**, **R₂**, **R₃** and **R₄** unabhängig voneinander für
(i) ein Wasserstoffatom oder
(ii) einen linearen oder verzweigten, cyclischen oder acyclischen C₁-C₅-Alkyl- oder -Alkenylrest, einen C₁-C₅-Alkoxyrest, iii) einen C₆-C₁₈-Arylrest, iv) einen 5- bis 8-gliedrigen heterocyclischen Rest; wobei diese Reste gegebenenfalls durch einen oder mehrere Reste, die aus den Resten: Hydroxyl, (C₁-C₄) Alkyl, (Di) (C₁-C₄) (alkyl)amino wie Dimethylamino, Carboxyl, Halogen, C₆-C₁₈-Aryl, Carboxamid und N-Methylcarboxamid ausgewählt sind, substituiert sein können;
stehen;
mit der Maßgabe, dass
- dann, wenn **R₁**, **R₂** und **R₃** für ein Wasserstoffatom stehen, **R₄** einen Carboxamid-, Methoxy-, Ethoxy-, 1,2,4-Triazolyl-, Cyclopentyl-, (C₁-C₆)Alkylcarbonyl- wie Acetyl-, (C₁-C₆)Alkoxycarbonyl- wie Methoxycarbonyl- oder Ethoxycarbonyl-, -C(O)-CH=CH-C(O)-OH-, gegebenenfalls durch ein Chloratom oder einen Hydroxylrest substituierten Phenyl-, Benzyl- oder 2,5-Dioxo-4-imidazolidinylrest bedeuten kann;
- dann, wenn **R₁** und **R₃** für ein Wasserstoffatom stehen, **R₂** für ein Wasserstoffatom oder einen Methyl- oder Ethylrest stehen kann und R₄ für einen Acetylrest stehen kann;
- dann, wenn **R₁**=**R₂**=H, **R₃** und **R₄** mit dem Stickstoffatom, das sie trägt, einen Piperidin-, 3-Methylpyrazol-, 3,5-Dimethylpyrazol- oder Maleinimidring bilden können;
- **R₁** und **R₂** sowie **R₃** and **R₄** mit dem Stickstoffatom, das sie trägt, einen Imidazolring bilden können;
- **R₅** and **R₆** unabhängig voneinander für
(iii) ein Wasserstoffatom oder
(iv) einen linearen oder verzweigten, cyclischen oder acyclischen C₁-C₅-Alkyl-, -Acyl- oder -Alkenylrest, einen C₁-C₅-Alkoxyrest, einen C₆-C₁₈-Arylrest, iv) einen 5- bis 8-gliedrigen heterocyclischen Rest; wobei diese Reste gegebenenfalls durch einen oder mehrere Reste, die aus den Resten: Hydroxyl, Amino, Dimethylamino, Carboxyl, Halogen, C₆-C₁₈-Aryl, Carboxamid und N-Methylcarboxamid ausgewählt sind, substituiert sein können;
stehen;
- **A** für einen zweiwertigen Rest steht, der aus den folgenden zweiwertigen Resten ausgewählt ist: -CH₂-CH₂-, -CH=CH-, -CH₂-C(O)-, -C(O)-N(H)-, -CH=N-, -C(O)-C(O)-, -CH(OH)-CH(OH)-, -[HO(O)C]CH-CH-, -CH(OH)-C(O) -, -CH₂-CH₂-CH₂-, -CH₂-N(H)-C(O)-, -CH=C(CH₃)-C(O)-, -N(H)-C(O)-N(H)-, -CH₂-CH₂-C(O)-, -CH₂-N(CH₃)-CH₂-, -N(H)-CH₂-N(H)-, -C(O)-CH(CH₃)- CH₂-, -C(O)-CH₂-C(O)-, -C(O)-N(H)-C(O)-, -C(O)-CH[C(O)OH)-CH₂-, -C(O)-CH=C[C(O)OH]-, -C(O)-CH=C(CH₃)-, -C(O)-C(NH₂)=CH-, -C(O)-C(CH₃)=N-, -C(O)-CH=CH-, -C(O)-CH=N- und -C(O)-N=CH-.

8. Verfahren nach einem der vorhergehenden Ansprüche, wobei der Harnstoff bzw. die Harnstoffderivate aus den folgenden Verbindungen, Salzen davon, Tautomeren oder Hydraten davon ausgewählt ist bzw. sind:
- Harnstoff
- Methylharnstoff
- Ethylharnstoff
- Propylharnstoff
- n-Butylharnstoff
- sec-Butylharnstoff
- Isobutylharnstoff
- tert-Butylharnstoff
- Cyclopentylharnstoff
- Ethoxyharnstoff
- Hydroxyethylharnstoff
- N-(2-Hydroxypropyl)harnstoff
- N-(3-Hydroxypropyl)harnstoff
- N-(2-Dimethylaminopropyl)harnstoff
- N-(3-Dimethylaminopropyl)harnstoff
- 1-(3-Hydroxyphenyl)harnstoff
- Benzylharnstoff
- N-Carbamoylmaleinamid
- N-Carbamoylmaleamidsäure
- Piperidinocarboxamid
- 1,2,4-Triazol-4-ylharnstoff
- Hydantoinsäure
- Methylallophanat
- Ethylallophanat
- Acetylharnstoff
- Hydroxyethylenharnstoff
- 2-(Hydroxyethyl)ethylenharnstoff
- Diallylharnstoff
- Chlorethylharnstoff
- N,N-Dimethylharnstoff
- N,N-Diethylharnstoff
- N,N-Dipropylharnstoff
- Cyclopentyl-l-methylharnstoff
- 1,3-Dimethylharnstoff
- 1,3-Diethylharnstoff
- 1,3-Bis(2-hydroxyethyl)harnstoff
- 1,3-Bis(2-hydroxypropyl)harnstoff
- 1,3-Bis(3-hydroxypropyl)harnstoff
- 1,3-Dipropylharnstoff
- Ethyl-3-propylharnstoff
- sec-Butyl-3-methylharnstoff
- Isobutyl-3-methylharnstoff
- Cyclopentyl-3-methylharnstoff
- N-Acetyl-N'-methylharnstoff
- Trimethylharnstoff
- Butyl-3,3-dimethylharnstoff
- Tetramethylharnstoff
- Benzylharnstoff
- Parabansäure
- 1,2-Dihydro-3-H-1,2,4-triazol-2-on
- Uracil
- 1-Methyluracil
- 3-Methyluracil
- 5-Methyluracil
- 1,3-Dimethyluracil
- 5-Azauracil
- 6-Azauracil
- 5-Fluoruracil
- 6-Fluoruracil
- 1,3-Dimethyl-5-fluoruracil
- 5-Aminouracil
- 6-Aminouracil
- 6-Amino-1-methyluracil
- 6-Amino-1,3-dimethyluracil
- 4-Chloruracil
- 5-Chloruracil
- 5,6-Dihydrouracil
- 5,6-Dihydro-5-methyluracil
- 2-Imidazolidon
- 1-Methyl-2-imidazolidinon
- 1,3-Dimethyl-2-imidazolidinon
- 4,5-Dihydroxyimidazolidin-2-on
- 1-(2-Hydroxyethyl)-2-imidazolidinon
- 1-(2-Hydroxypropyl)-2-imidazolidinon
- 1-(3-Hydroxypropyl)-2-imidazolidinon
- 4,5-Dihydroxy-1,3-dimethylimidazolidin-2-on
- 1,3-Bis(2-hydroxyethyl)-2-imidazolidinon
- 2-Imidazolidon-4-carbonsäure
- 1-(2-Aminoethyl)-2-imidazol
- 4-Methyl-1,2,4-triazolin-3,5-dion
- 2,4-Dihydroxy-6-methylpyrimidin
- 1-Amino-4,5-dihydro-1H-tetrazol-5-on
- Hydantoin
- 1-Methylhydantoin
- 5-Methylhydantoin
- 5,5-Dimethylhydantoin
- 5-Ethylhydantoin
- 5-n-Propylhydantoin
- 5-Ethyl-5-methylhydantoin
- 5-Hydroxy-5-methylhydantoin
- 5-Hydroxymethylhydantoin
- 1-Allylhydantoin
- 1-Aminohydantoin
- Hydantoin-5-essigsäure
- 4-Amino-1,2,4-triazolon-3,5-dion
- Hexahydro-1,2,4,5-tetrazin-3,6-dion
- 5-Methyl-1,3,5-triazinon-2-on
- 1-Methyltetrahydropyrimidin-2-on
- 2,4-Dioxohexahydro-1,3,5-triazin
- Urazol
- 4-Methylurazol
- Orotsäure
- Dihydroxyorotsäure
- 2,4,5-Trihydroxypyrimidin
- 2-Hydroxy-4-methylpyrimidin
- 4,5-Diamino-2,6-dihydroxypyrimidin
- Barbitursäure
- 1,3-Dimethylbarbitursäure
- Cyanursäure
- 1-Methylhexahydropyrimidin-2,4-dion
- 1,3-Dimethyl-3,4,5,6-tetrahydro-2-1H-pyrimidinon
- 5-(Hydroxymethyl-2,4-(1H,3H)-pyrimidindion
- 2,4-Dihydroxypyrimidin-5-carbonsäure
- 6-Azathymin
- 5-Methyl-1,3,5-triazinan-2-on
- N-Carbamoylmaleamidsäure und
- Alloxanmonohydrat.

9. Verfahren nach einem der vorhergehenden Ansprüche, wobei das aminbildende Derivat bzw. die aminbildenden Derivate der Formel **(II)** aus Harnstoff, Hydroxyethylharnstoff, Salzen davon und Hydraten davon und spezieller Harnstoff, Salzen davon und Hydraten davon ausgewählt ist bzw. sind.

10. Verfahren nach einem der vorhergehenden Ansprüche, **dadurch gekennzeichnet, dass** das aminbildende Derivat bzw. die aminbildenden Derivate der Formel **(II)**, Salze davon und Hydrate davon in einem Aktivmaterialgehalt im Bereich von 0,01 bis 15 Gew.-%, vorzugsweise 0,1 bis 10 Gew.-%, vorzugsweise im Bereich von 0,2 bis 5 Gew.-%, bezogen auf das Gesamtgewicht der Zusammensetzung, vorliegt bzw. vorliegen.

11. Verfahren nach einem der vorhergehenden Ansprüche, wobei das Gewichtsverhältnis von Dicarbonylderivaten der Formel **(I)** und/oder Derivaten davon und/oder Hydratformen davon und/oder Salzen davon zu aminbildenden Derivaten der Formel **(II)** oder Hydratformen davon und/oder Salzen davon im Bereich von 0,1 bis 100 und besser von 1 bis 20 liegt.

12. Verfahren nach einem der vorhergehenden Ansprüche, **dadurch gekennzeichnet, dass** die Zusammensetzung wässrig ist und Wasser in einer Konzentration, die vorzugsweise im Bereich von 5 bis 98 Gew.-%, besser von 5 bis 90 Gew.-%, noch besser von 10 bis 90 Gew.-%, bezogen auf das Gesamtgewicht der Zusammensetzung, liegt, umfasst.

13. Verfahren nach einem der vorhergehenden Ansprüche, **dadurch gekennzeichnet, dass** die Zusammensetzung einen pH-Wert von weniger als 4 aufweist, wobei der pH-Wert vorzugsweise im Bereich von 1 bis 3, besser von 1,5 bis 3, noch besser von 1,7 bis 3, liegt.

14. Verfahren nach einem der vorhergehenden Ansprüche, **dadurch gekennzeichnet, dass** sich die Zusammensetzung aus dem Mischen von mehreren Zusammensetzungen ergibt.

15. Verfahren nach einem der vorhergehenden Ansprüche, wobei auf das Aufbringen eine Kontaktzeit im Bereich von 10 bis 60 Minuten folgt.

## Claims

1. Method for straightening keratin fibres, in particular hair, which comprises the application to the hair of a cosmetic composition comprising
- one or more dicarbonyl compounds corresponding to formula (I) below and/or derivatives thereof and/or hydrates thereof and/or salts thereof, the derivatives being chosen from esters of the carboxyl group(s), amides of the carboxyl group(s), and (thio)acetals and hemi(thio)acetals of the carbonyl function(s) of the compounds or formula **(I)** in free form or optionally in salt or hydrate form, preferably in free or hydrate form: 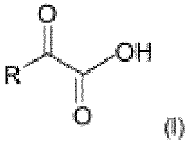 in which formula (I)
R represents an atom or group chosen from i) hydrogen, ii) carboxyl -C(O)OH, iii) linear or branched C₁-C₆ alkyl, optionally substituted preferably with at least one hydroxyl -OH, carboxyl -C(O)-OH or halogen, such as Br, radical; iv) optionally substituted phenyl, v) optionally substituted benzyl, iv) and v) preferably being optionally substituted with at least one -OH or -C(O)OH radical; vi) an indolyl radical and vii) an imidazolylmethyl radical and its tautomers such as 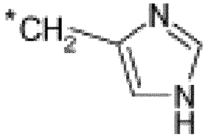 with * representing the part linked to the rest of the molecule,
- one or more amine-generating derivatives chosen from urea or urea derivatives, salts thereof or hydrates thereof, the urea derivatives being chosen from compounds, other than urea, comprising, in their chemical formula, a carbonyl group, simply bonded to two nitrogen atoms, that is to say a unit 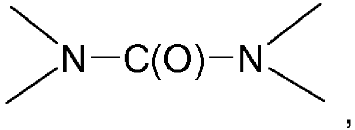 followed by a straightening step by means of a straightening iron at a temperature of at least 150°C, preferably ranging from 150 to 250°C.

2. Method according to Claim 1 in which R represents i) a hydrogen atom or ii) a linear or branched C₁-C₆ alkyl group optionally substituted with a carboxyl group.

3. Method according to either one of the preceding claims, in which the dicarbonyl compound(s) corresponding to formula (I) and/or derivatives thereof and/or hydrates thereof and/or salts thereof is (are) chosen from glyoxylic acid, pyruvic acid, one of their derivatives, their salts and their hydrates, preferably from glyoxylic acid, one if its derivatives and the hydrate forms of these compounds.

4. Method according to any one of the preceding claims, in which the dicarbonyl derivative(s) corresponding to formula (I) and/or derivatives thereof is (are) chosen from glyoxylic acid esters, glyoxylic acid amides, glyoxylic acid (thio)acetals and hemi(thio)acetals, and glyoxylic acid ester (thio)acetals and hemi(thio)acetals.

5. Method according to Claim 3, in which the glyoxylic acid is in its hydrate form.

6. Method according to any one of Claims 1 to 5, in which the composition comprises from 0.5% to 15% by weight of one or more dicarbonyl derivatives corresponding to formula (I) and/or derivatives thereof and/or hydrates thereof and/or salts thereof, preferably from 3% to 15%, preferentially from 5% to 10% by weight of the total weight of the composition.

7. Method according to any one of the preceding claims, in which the urea or the urea derivatives are chosen from the compounds of formula (a) or (b), salts thereof, tautomers thereof, or hydrates thereof: 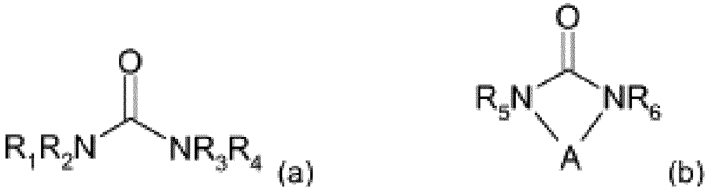 in which formulae (a) and (b):
- **R₁**, **R₂**, **R₃** and **R₄** represent, independently of one another:
(i) a hydrogen atom or
(ii) a linear or branched, cyclic or acyclic C₁-C₅ alkyl or alkenyl radical or a C₁-C₅ alkoxy radical, iii) a C₆-C₁₈ aryl radical, iv) a heterocyclic radical having from 5 to 8 ring members; these radicals being optionally substituted with one or more radicals chosen from the radicals: hydroxyl, (C₁-C₄) alkyl, (di) (C₁-C₄) (alkyl) amino such as dimethylamino, carboxyl, halogen, C₆-C₁₈ aryl, carboxamide and N-methylcarboxamide;
it being understood that
- when **R₁**, **R₂** and **R₃** represent a hydrogen atom, R₄ can denote a carboxamide, methoxy, ethoxy, 1,2,4-triazolyl or cyclopentyl radical, a (C₁-C₆)alkylcarbonyl radical such as an acetyl radical, a (C₁-C₆)alcoxycarbonyl radical such as a methoxycarbonyl or ethoxycarbonyl radical, a -C(O)-CH=CH-C(O)-OH radical, a phenyl radical optionally substituted with a chlorine atom or a hydroxyl, benzyl or 2,5-dioxo-4-imidazolidinyl radical;
- when **R₁ and R₃** represent a hydrogen atom, **R₂** can represent a hydrogen atom, a methyl radical or ethyl radical and R₄ can represent an acetyl radical;
- when **R₁=R₂**=H, **R₃** and **R₄** can form, with the nitrogen atom which bears them, a piperidine, 3-methylpyrazole, 3,5-dimethylpyrazole or maleimide ring;
- **R₁** and **R₂**, and also **R₃** and **R₄**, can form, with the nitrogen atom that bears them, an imidazole ring;
- **R₅** and **R₆** represent, independently of one another,
(iii) a hydrogen atom or
(iv) a linear or branched, cyclic or acyclic C₁-C₅ lower alkyl, acyl or alkenyl radical, a C₁-C₅ alkoxy radical, a C₆-C₁₈ aryl radical, a heterocyclic radical having from 5 to 8 ring members; these radicals being optionally substituted with a radical chosen from the radicals: hydroxyl, amino, dimethylamino, carboxyl, halogen, C₆-C₁₈ aryl, carboxamide and N-methylcarboxamide;
- **A** is a divalent radical chosen from the following divalent radicals: -CH₂-CH₂-,-CH=CH-,-CH₂-C(O)-, -C(O)-N(H)-, -CH=N-, -C(O)-C(O)-, -CH(OH)-CH(OH)-, -[HO(O)C]CH-CH-, -CH(OH)-C(O)-, -CH₂-CH₂-CH₂-, -CH2-N(H)-C(O)-, -CH=C(CH₃)-C(O)-, -N(H)-C(O)-N(H)-, -CH₂-CH₂-C(O)-, -CH₂-N(CH₃)-CH₂-, -N(H)-CH₂-N(H)-, -C(O)-CH(CH₃)-CH₂-, -C(O)-CH₂-C(O)-, -C(O)-N(H)-C(O)-,-C(O)-CH[C(O)OH]-CH₂, -C(O)-CH=C[C(O)OH]-, -C(O)-CH=C(CH₃)-, -C(O)-C(NH₂)=CH-, -C(O)-C(CH₃)=N-, -C(O)-CH=CH-, -C(O)-CH=N- and -C(O)-N=CH-.

8. Method according to the preceding claim, in which the urea or the urea derivatives are chosen from the following compounds, salts thereof, tautomers thereof or hydrates thereof:
- urea
- methylurea
- ethylurea
- propylurea
- n-butylurea
- sec-butylurea
- isobutylurea
- tert-butylurea
- cyclopentylurea
- ethoxyurea
- hydroxyethylurea
- N-(2-hydroxypropyl)urea
- N-(3-hydroxypropyl)urea
- N-(2-dimethylaminopropyl)urea
- N-(3-dimethylaminopropyl)urea
- 1-(3-hydroxyphenyl)urea
- benzylurea
- N-carbamoyl maleamide
- N-carbamoyl maleamic acid
- piperidinocarboxamide
- 1,2,4-triazol-4-ylurea
- hydantoic acid
- methyl allophanate
- ethyl allophanate
- acetylurea
- hydroxyethyleneurea
- 2-(hydroxyethyl)ethyleneurea
- diallylurea
- chloroethylurea
- N,N-dimethylurea
- N,N-diethylurea
- N,N-dipropylurea
- cyclopentyl-1-methylurea
- 1,3-dimethylurea
- 1,3-diethylurea
- 1,3-bis(2-hydroxyethyl)urea
- 1,3-bis(2-hydroxypropyl)urea
- 1,3-bis(3-hydroxypropyl)urea
- 1,3-dipropylurea
- ethyl-3-propylurea
- sec-butyl-3-methylurea
- isobutyl-3-methylurea
- cyclopentyl-3-methylurea
- N-acetyl-N'-methylurea
- trimethylurea
- butyl-3,3-dimethylurea
- tetramethylurea and
- benzylurea,
- parabanic acid
- 1,2-dihydro-3-H-1,2,4-triazol-2-one
- uracil
- 1-methyluracil
- 3-methyluracil
- 5-methyluracil
- 1,3-dimethyluracil
- 5-azauracil
- 6-azauracil
- 5-fluorouracil
- 6-fluorouracil
- 1,3-dimethyl-5-fluorouracil
- 5-aminouracil
- 6-aminouracil
- 6-amino-1-methyluracil
- 6-amino-1,3-dimethyluracil
- 4-chlorouracil
- 5-chlorouracil
- 5,6-dihydrouracil
- 5,6-dihydro-5-methyluracil
- 2-imidazolidone
- 1-methyl-2-imidazolidinone
- 1,3-dimethyl-2-imidazolidinone
- 4,5-dihyroxyimidazolidin-2-one
- 1-(2-hydroxyethyl)-2-imidazolidinone
- 1-(2-hydroxypropyl)-2-imidazolidinone
- 1-(3-hydroxypropyl)-2-imidazolidinone
- 4,5-dihydroxy-1,3-dimethylimidazolidin-2-one
- 1,3-bis(2-hydroxyethyl)-2-imidazolidinone
- 2-imidazolidone-4-carboxylic acid
- 1-(2-aminoethyl)-2-imidazole
- 4-methyl-1,2,4-triazoline-3,5-dione
- 2,4-dihydroxy-6-methylpyrimidine
- 1-amino-4,5-dihydro-1H-tetrazol-5-one
- hydantoin
- 1-methylhydantoin
- 5-methylhydantoin
- 5,5-dimethylhydantoin
- 5-ethylhydantoin
- 5-n-propylhydantoin
- 5-ethyl-5-methylhydantoin
- 5-hydroxy-5-methylhydantoin
- 5-hydroxymethylhydantoin
- 1-allylhydantoin
- 1-aminohydantoin
- hydantoin-5-acetic acid
- 4-amino-1,2,4-triazolone-3,5-dione
- hexahydro-1,2,4,5-tetrazine-3,6-dione
- 5-methyl-1,3,5-triazinon-2-one
- 1-methyltetrahydropyrimidin-2-one
- 2,4-dioxohexahydro-1,3,5-triazine
- urazole
- 4-methylurazole
- orotic acid
- dihydroxyorotic acid
- 2,4,5-trihydroxypyrimidine
- 2-hydroxy-4-methylpyrimidine
- 4,5-diamino-2,6-dihydroxypyrimidine
- barbituric acid
- 1,3-dimethylbarbituric acid
- cyanuric acid
- 1-methylhexahydropyrimidine-2,4-dione
- 1,3-dimethyl-3,4,5,6-tetrahydro-2-1H-pyrimidinone
- 5-hydroxymethyl-2,4-(1H,3H)-pyrimidinedione
- 2,4-dihydroxypyrimidine-5-carboxylic acid
- 6-azathymine
- 5-methyl-1,3,5-triazinan-2-one
- N-carbamoylmaleamic acid and
- alloxane monohydrate.

9. Method according to any one of the preceding claims, in which the amine-generating derivative(s) corresponding to formula **(II)** is (are) chosen from urea, hydroxyethylurea, salts thereof and hydrates thereof, and more particularly urea and salts thereof, and hydrates thereof.

10. Method according to any one of the preceding claims, **characterized in that** the amine-generating derivative(s) corresponding to formula **(II)**, salts thereof and hydrates thereof are present in a content of active material ranging from 0.01% to 15% by weight, preferably from 0.1% to 10% by weight, preferably ranging from 0.2% to 5% by weight relative to the total weight of the composition.

11. Method according to any one of the preceding claims, in which the weight ratio of dicarbonyl derivatives corresponding to formula **(I)** and/or a derivative thereof and/or a hydrate form thereof and/or salts thereof/amine-generating derivatives corresponding to formula **(II)** or hydrate forms thereof and/or salts thereof ranges from 0.1 to 100, better still from 1 to 20.

12. Method according to any one of the preceding claims, **characterized in that** the composition is aqueous and comprises water in a concentration preferably ranging from 5% to 98%, better still from 5% to 90%, even better still from 10% to 90% by weight relative to the total weight of the composition.

13. Method according to any one of the preceding claims, **characterized in that** the composition has a pH of less than 4, the pH preferentially ranging from 1 to 3, better still from 1.5 to 3, even better still from 1.7 to 3.

14. Method according to any one of the preceding claims, **characterized in that** the composition results from the mixing of several compositions.

15. Method according to any one of the preceding claims, in which the application of the composition is followed by a contact time ranging from 10 to 60 minutes.
